# EUROPEAN PATENT APPLICATION

(11) **EP 2 119 445 A1**
(43) Date of publication of application: **18.11.2009**
(21) Application number: 08710936.9
(22) Date of filing: 07.02.2008
(51) Int. Cl.: A61K 35/20, A61K 38/16, A61K 38/44, A61P 3/10, A61P 19/02, A61P 29/00, A61P 37/02

(54) **PROPHYLACTIC AGENT FOR AUTOIMMUNE DISEASE**

(30) Priority: 08.02.2007 JP 2007028809
(71) Applicant: Snow Brand Milk Products Co., Ltd., Sapporo-shi Hokkaido 065-0043 (JP)
(72) Inventor: KAWAKAMI, Hiroshi, Kawagoe-shi Saitama 350-1142 (JP)
(74) Representative: Wakerley, Helen Rachael
(86) International application number: PCT/JP2008/052057
(87) International publication number: WO 2008/096826

(57) **Abstract**

Provided is a prophylactic agent for an autoimmune disease, comprising a fraction of a milk-derived basic protein, lactoperoxidase, and lactoferrin as active ingredients. The agent can be taken on a daily basis, and even if it is taken over a long period of time, its safety is high, and hence, an autoimmune disease such as type I diabetes mellitus or rheumatoid arthritis, which could not be effectively prevented or treated by a conventional method, can be prevented.

## Description

### Technical Field

The present invention relates to a prophylactic agent for an autoimmune disease, including a fraction of a milk-derived basic protein as an active ingredient. The present invention also relates to a prophylactic agent for an autoimmune disease, **characterized in that** the milk-derived basic protein(s) is lactoperoxidase and/or lactoferrin. The present invention also relates to a prophylactic agent for an autoimmune disease, **characterized in that** a fraction of a milk-derived basic protein, and lactoperoxidase and/or lactoferrin are included as active ingredients, and that the autoimmune disease is type I diabetes mellitus or rheumatoid arthritis. By orally ingesting the fraction of a milk-derived basic protein, lactoperoxidase, and lactoferrin of the present invention, it is possible to prevent autoimmune diseases such as type I diabetes mellitus and rheumatoid arthritis, which could not be effectively prevented or treated by conventional methods.

### Background Art

The living body always distinguishes "self" from "nonself' in the thymus and controls so as not to cause excessive immune response to "self". The phenomenon where, in the thymus, clones responding to "self" are dead and only clones responding to "nonself" survive is referred to as clonal selection. The state where the living body does not respond to "self" any more is referred to as self tolerance. However, it does not mean that the immune response to the self dose not occur at all. Sensitized lymphocytes which recognize a trace amount of an autoantibody or a trace amount of an autoantigen is always present even in a normal individual. The reaction is called autoimmunity. That is, the autoimmunity is a physiological reaction always occurring in the living body. On the other hand, if the self tolerance is destroyed due to a genetic factor, an environmental factor, or the like, excessive immune response to the self occurs so that a large amount of the autoantibody is produced. In addition, autosensitized lymphocyte clones amplify, resulting in morbidity. Thus, the pathosis generated due to disturbance of the immune control mechanism is the autoimmune disease.

The autoimmune diseases includes a case where a problematic autoantigen is localized in a specific organ, tissue, or cell, and only the organ is hurt. The case is referred to as an organ-specific autoimmune disease. On the other hand, an autoimmune disease, in which an autoantibody with respect to an autoantigen evenly present in the whole body such as DNA is demonstrated, and a systemic pathology such as vasculitis occurs, is called a systemic autoimmune disease. Examples of the organ-specific autoimmune disease include diseases such as autoimmune hemolytic anemia, idiopathic thrombocytopenic purpura, autoimmune thyroiditis, myasthenia gravis, multiple sclerosis, type I diabetes mellitus and the like. In those diseases, an autoantibody with respect to an antigen component in a pathological organ is recognized, and infiltration of a lymphocyte, phlogocyte, and histiocyte, and formation of a germinal center, and the like are observed histopathologically. Examples of the systemic autoimmune disease include systemic lupus erythematosus (SLE), rheumatoid arthritis (RA) and the like. An anti-nuclear (DNA) antibody appears in SLE, and a rheumatoid factor appears in RA. The rheumatoid factor is an autoantibody with respect to Fc part of IgG.

A background technology of type I diabetes mellitus involved in the autoimmunity is described. Insulin also used for the treatment of diabetes mellitus is an important hormone for homeostasis of blood glucose level and produced in islets of Langerhans of the pancreas. The human autoimmune type I insulin-dependent diabetes mellitus (IDDM) is characterized by progressive autoimmune destruction of pancreas β cells in the islets of Langerhans by autoreactive T cells and antibodies. The destruction process may be generated through destroy of peripheral tolerance or a defective clone-elimination mechanism. A non-obese diabetic (NOD) mouse is a classic mouse model which naturally develops autoimmune type I IDDM having an immunopathological profile similar to that of human IDDM (see Non-patent Document 1). The development of IDDM in both the mouse and the human is under the control of polygene. IDDM is caused by destruction of a pancreatic islet cell (β cell) mediated by CD4, CD8, and a macrophage (see Non-patent Documents 2, 3, and 4). In addition, it is reported that the destruction of the β cell is mediated by MHC-dependency cytotoxicity, and a β cell autoantigen-specific T cell is involved in the cause of IDDM (see Non-patent Documents 5, 6, and 7). At present, it is assumed that T cell autoreactivity depends on peripheral activation of a regulatory T cell due to a defect of the thymus and/or after a change in cytokine production (see Non-patent Documents 8, 9, 10, 11, and 12).

The sensitivities of the human and the NOD mouse to IDDM are strongly associated with the expression of MHC class II β-chain lacking in a general aspartic acid residue at position 57 (Asp-57) (see Non-patent Document 13). Actually, it is reported that the expression of transgenic class II β-chain containing Asp-57 protects the NOD mouse from spontaneous onset of IDDM (see Non-patent Documents 14, 15, 16, 17, and 18). Induction of a regulatory T cell may be associated with the experimental autoimmune encephalitis (EAE), which is developed after recovery from acute symptom expression (see Non-patent Documents 19 and 20). There is a finding suggesting that a suppressor group is a Th1 type (see Non-patent Document 21). However, there are many unexplained parts about the exact type and properties of those cells. The above-mentioned induction of the regulatory T cell by systemic administration of a cytokine which mediates wide immunosuppression has been attempted, but the treatment involving the attempt is too non-specific and often accompanies harmful side effects.

Arthritis is also developed by autoimmunity. The adjuvant arthritis in rats is induced by inoculation with various mycobacteria. Such induced arthritis can be suppressed by administration of *Mycobacterium bovis* of bovines, i.e., hsp65 of BCG (also referred to as 64kD antigen A) (see Patent Documents 1 and 2). BCG hsp65 is identical in amino acid sequence to hsp65 of human *Mycobacterium tuberculosis* (see Non-patent Document 22), and there is disclosed methods of treatment or prophylaxis of "arthritis-type autoimmune disease" by using this protein. That is, the use of human *Mycobacterium tuberculosis* hsp65 (also referred to as hsp60) in the treatment of IDDM in the NOD mouse is reported (see Patent Document 2 and Non-patent Document 23). However, the report is limited to the research of hsp65 which responds to one unique autoantigen and is not the research of an immunomodulator. In addition, it is confirmed that the method cannot be applied to other stress proteins (see Non-patent Document 23). There is also reported further research using peptide p277, which is presumed to contain an epitope which is a fragment of human hsp60 and corresponds to an important epitope of human *Mycobacterium tuberculosis* hsp65 (see Patent Document 3 and Non-patent Document 24). Patent Document 3 suggests that the human hsp60 protein can be used in the treatment of IDDM "therapeutically", but data using the human hsp60 protein was not exhibited at all. A general problem accompanying the attempt of inducing tolerance to the autoantigen of a specific disease is that before such tolerance is achieved or instead of achievement of such tolerance, the administration of an autoantigen has a possibility of inducing the disease by increasing destructive immune response against a target tissue. For example, the administration of human *Mycobacterium tuberculosis* hsp65 or p277 may cause a monophasic hyperglycemia prior to the protection (see Patent Document 3 and Non-patent Document 25). Accordingly, there is a risk of aggravation of a disease at least for a short period, the disease being derived from the administration of an autoantigen, and thus, the usability of the autoantigen is problematic. Further, at least 12 specific autoantigens which are targets of IDDM autoimmune response and peptides thereof are present (see Non-patent Document 26). The treatment using p277 alone may be thought not to treat a disease also associated with another autoantigen. An effective treatment using a peptide autoantigen as an immunogen may include necessarily identification of a specific antigen or an antigen set which is a target of a specific IDDM patient. Therefore, the approaches mentioned in those researches, even though it is the best one of them, are too general (for example, systemic administration of a cytokine) or too specific to provide a practical and effective treatment for an autoimmune disease.

In the treatment for other autoimmune diseases, a corticosteroid preparation, an immunosuppressive agent, monoclonal antibody (such as anti-CD3 antibody, anti-TNF-a antibody), a soluble-type cytokine receptor (soluble-type TNF-α receptor), and the like are used (see Non-patent Document 27).
The corticosteroid preparation inhibits the production of an inflammatory cytokine such as IL-1 or TNF-α, further suppresses strongly proliferation reaction of a T cell and antigen production from a B cell. In addition, the corticosteroid preparation also suppresses inflammatory cell infiltration through the suppression of expression of adhesion molecules such as E-selectin and ICAM-1. Both cyclosporine and tacrolimus among antibiotics bind to a receptor in the cell, the receptor being collectively called immunophilin. The drug bound to immunophilin further forms a complex with calcineurin as a calcium-dependent phosphatase, to thereby inhibit the phosphatase activity. As a result, NFAT (nuclear factor of activated T cell) as a transcription factor cannot be dephosphorylated, and NFAT cannot transfer from the inside of the cell to the inside of the nuclear, whereby transcription of the cytokine gene of a such as IL-2 is inhibited. The anti-TNF-α monoclonal antibody neutralizes TNF-α as a cytokine involved in the inflammatory reaction at the local site of lesion of chronic rheumatoid arthritis and Crohn's disease to thereby suppress the inflammatory reaction. In addition, the antibody suppresses the progress of osteolysis in an arthrosis, which influences the functional prognosis of a patient in RA. The soluble-type TNF-α receptor is a biological preparation obtained by fusing the extracellular domain of p75 molecules of a TNF-α receptor and the Fc part of human IgG1 and expressing the resultant as a dimer in a Chinese hamster ovary cell. The soluble-type TNF-α receptor is used in a treatment targeting TNF-α as well as the anti-TNF-α monoclonal antibody. [0008]
However, those substances are drugs themselves and are far from having high safety as well as administration of an autoantigen as described above. In order to prevent or treat the autoimmune disease as described above, there is earnestly demanded, rather than ingestion of those drugs, the development of a moderate prophylactic agent for an autoimmune disease obtained from a substance which can be ingested routinely, has no problem even when ingested over a long period, and can be used as a food material.
[Patent Document 1] USP No.5,354,691
[Patent Document 2] USP No.5,268,170
[Patent Document 3] USP No.5,578,303
[Non-patent Document 1] Makino, S. et al., 1985, Current Topics in Clinical and Experimental Aspects of Diabetes (Elsevier: Amsterdam)
[Non-patent Document 2] Castano and Eisenbarth, 1990, Ann. Rev. Immunol., 8: 647-79
[Non-patent Document 3] Haskins et al., 1990, Science, 249: 1433-36
[Non-patent Document 4] Nakano et al., 1991, J. Exp. Med., 173: 1091-7
[Non-patent Document 5] Reich et al., 1993, Nature, 341: 326-9
[Non-patent Document 6] Tisch et al., 1993, Nature, 366: 72-5
[Non-patent Document 7] Kaufmen et al., 1993, Nature, 366: 69-72
[Non-patent Document 8] Serreze et al., 1988, J. Immunol., 140: 3801
[Non-patent Document 9] Serreze et al., 1993, J. Immunol., 150: 2534
[Non-patent Document 10] Serreze et al., 1993, Proc. Natl. Acad. Sci. USA, 90: 9625
[Non-patent Document 11] Zipris et al., 1991, J. Immunol., 146: 3763
[Non-patent Document 12] Rapoport et al., 1993, J. Exp. Med., 178: 87
[Non-patent Document 13] Todd, J.A., 1990, Immunol. Today, 11: 122-9
[Non-patent Document 14] Nishimoto et al., 1987, Nature, 328: 432-4
[Non-patent Document 15] Bohme et al., 1990, Science, 249: 293-5
[Non-patent Document 16] Miyazaki et al., 1990, Nature, 345: 722-4
[Non-patent Document 17] Slattery et al., 1990, Nature, 345: 724-6
[Non-patent Document 18] Singer et al., 1993, Proc. Natl. Acad. Sci. USA, 90: 9566-70
[Non-patent Document 19] Hamaguchi and Leiter, 1990, Diabetes, 39: 415
[Non-patent Document 20] Lider et al., 1988, Science, 239: 181
[Non-patent Document 21] Tan et al., 1995, J. Exp. Med., 182: 87-97
[Non-patent Document 22] Shinnick et al., 1987, Infect. Immun., 55: 1932-1935
[Non-patent Document 23] Elias et al., 1990, Proc. Natl. Acad. Sci. USA, 87: 1576-1580
[Non-patent Document 24] Elias and Cohen, 1995 Diabetes, 44: 1132-1138
[Non-patent Document 25] Elias et al., 1995, Eur. J. Immunol., 25: 2851-2857
[Non-patent Document 26] Solimena and De Camilli, 1996, Nature Medicine, 2: 1311
[Non-patent Document 27] Saishin Igaku (Current Medicine), Vol. 61, No. 5, 917-1009, 2006

### Disclosure of the Invention

### Problems to be solved by the Invention

An object of the present invention is to provide a substance which can be ingested routinely and has an autoimmune disease-preventing effect with high safety even when ingested over a long period of time. Further, another object of the present invention is to provide a substance which prevents or treats diseases caused by autoimmunity, such as type I diabetes mellitus and rheumatoid arthritis, which could not be effectively prevented or treated by conventional methods.

### Means for solving the Problems

The inventors of the present invention have continued to search substances having an autoimmune disease-preventing effect and being present in milk in order to obtain a substance which prevents or treats an autoimmune disease. As a result, the inventors have found that a basic protein present in milk in only a trace amount has the autoimmune disease-preventing effect, and have found that a fraction of the milk-derived basic protein can be used as an active ingredient of a prophylactic agent for an autoimmune disease, thereby completing the present invention.
That is, the present invention relates to a prophylactic agent for an autoimmune disease, comprising a fraction of a milk-derived basic protein as an active ingredient. Further, the present invention relates to a prophylactic agent for an autoimmune disease, wherein the milk-derived basic protein comprises lactoperoxidase and/or lactoferrin. Further, the present invention relates to a prophylactic agent for an autoimmune disease, comprising a fraction(s) of a milk-derived basic protein(s), lactoperoxidase and/or lactoferrin, as an active ingredient(s), wherein the autoimmune disease is type I diabetes mellitus or rheumatoid arthritis. By oral ingestion of the fraction of the milk-derived basic protein, lactoperoxidase, and lactoferrin, according to the present invention, it is possible to prevent the autoimmune diseases such as type I diabetes mellitus and rheumatoid arthritis, which could not be effectively prevented or treated by conventional methods.

### Effects of the Invention

The prophylactic agent for an autoimmune disease of the present invention can prevent an autoimmune disease by administration thereof, thereby being useful in the treatment and prevention of diseases caused by autoimmunity, such as type I insulin-dependent diabetes mellitus or rheumatoid arthritis.

### Best Mode for carrying out the Invention

A prophylactic agent for an autoimmune disease of the present invention is characterized by including a fraction of a milk-derived basic protein as an active ingredient. Further, the prophylactic agent for an autoimmune disease is **characterized in that** the milk-derived basic protein includes lactoperoxidase and/or lactoferrin. Further, the prophylactic agent for an autoimmune disease is characterized by including a fraction(s) of a milk-derived basic protein(s), lactoperoxidase and/or lactoferrin, as an active ingredient(s), and is **characterized in that** the autoimmune disease is type I diabetes mellitus or rheumatoid arthritis.

The fraction of a milk-derived basic protein, lactoperoxidase, and lactoferrin in the present invention are prepared from milk of mammalians. As the source thereof, milk of cows, buffaloes, humans, pigs, sheep, goats, and horses, and the like are exemplified.

As a method of obtaining a fraction of a milk-derived basic protein as an active ingredient of the prophylactic agent for an autoimmune disease of the present invention, a method of adsorbing a basic protein by bringing milk or a milk-derived raw material to a cation exchanger, and obtaining a fraction of a milk-derived basic protein by eluting the fraction of the basic protein adsorbed on the cation exchanger with an elution solution having a pH exceeding 5 and an ion intensity exceeding 0.5 (JP 05-202098 A), a method of obtaining a fraction of a milk-derived basic protein by using an arginic acid gel (JP 61-246198 A), a method of obtaining from whey by using an inorganic porous particles (JP 01-86839 A), a method of obtaining from milk by using a sulfated ester compound (JP 63-255300 A), and the like are known. In the present invention, the fraction of a milk-derived basic protein obtained by those methods can be used.

The fraction of a milk-derived basic protein of the present invention has the following properties:
1) the fraction of a milk-derived basic protein consists of several kinds of proteins each having a molecular weight in the range of 3,000 to 80,000 determined by sodium dodecyl sulfate-polyacrylamide electrophoresis (SES-PAGE);
2) the fraction of a milk-derived basic protein contains 95 wt% or more of protein and contains a little amount of fat and ash;
3) the protein is mainly lactoferrin and lactoperoxidase; and
4) the amino acid composition of the protein contains 15 wt% or more of basic amino acids such as lysine, histidine, and arginine.

Lactoperoxidase and lactoferrin as active ingredients of the prophylactic agent for an autoimmune disease of the present invention are known substances and are available in the market. For producing lactoperoxidase and lactoferrin, known methods, e.g., a method of purifying lactoperoxidase and lactoferrin by using a sulfonated carrier (JP-A-H03-109400), can be industrially and advantageously used.

When administrating the prophylactic agent for an autoimmune disease of the present invention, the fraction of a milk-derived basic protein, lactoperoxidase, and lactoferrin as active ingredients can be used as they are, and those ingredients can be used by preparing a powder, a granule, a tablet, a capsule, a drinkable preparation or the like according to a conventional method.

In the present invention, an oral preparation such as a powder, a granule, a tablet, a capsule or the like can be prepared according to a conventional method by using, for example, starch, lactose, saccharose, mannite, carboxymethyl cellulose, corn starch, inorganic salts, and the like. In addition to the above diluting agent, a binder, a disintegrator, a surfactant, a lubricant, a fluidity accelerator, a coloring agent, a flavor, or the like can be appropriately used in the preparations.

Further, those decomposed substances of lactoferrin and lactoferrin are added to nutritive substances, drinks and foods, and the like, as they are or after prepared, whereby prevention of an autoimmune disease can be attempted. Note that because the fraction of a milk-derived basic protein is relatively stable to heat, a raw material containing the fraction of a milk-derived basic protein can also be sterilized by heating under conditions usually taken.

The dosage of the prophylactic agent for an autoimmune disease of the present invention is different depending on age, treatment effect, pathological condition, and the like. According to the results of the animal experiment using mice, it was revealed that 20 mg or more of the fraction of a milk-derived basic protein per 1 kg of mouse weight must be administered for exhibiting the autoimmune disease-preventing effect. For this reason, according to an extrapolation (Sequel to Development of Drugs, written by Hajime Yasuhara and Shinichi Kobayashi, Vol. 8, 7 to 18, Hirokawa-Shoten, Ltd. 1991), the effective dosage in humans is 20 mg or more per adult per day. Therefore, the fraction of a milk-derived basic protein may be administered so as to keep the necessary amount.

Next, the present invention is described in detail with reference to examples and test examples. However, those merely illustrate embodiments of the present invention and the present invention is not limited by the examples and test examples.

### Example 1

After a column (diameter 5 cm × height 30 cm) filled with 400 g of sulfonated Chitopearl (manufactured by Fuji Spinning Co., Ltd.) as a cation exchange resin was washed with deionized water sufficiently, 40 1 of unsterilized defatted milk (pH 6.7) were passed through the column at a flow rate of 25 ml/min. After that, the column was washed with deionized water sufficiently, and a fraction of a basic protein that adhered to the resin was eluted with a 0.02 M carbonate buffer (pH 7.0) containing 0.98 M sodium chloride. Then, the eluate was desalted with a reverse osmotic (OS) membrane and concentrated. After that, the resultant was freeze-dried, thereby obtaining 21 g of a powdered fraction of a milk-derived basic protein which is an active ingredient of a prophylactic agent for an autoimmune disease of the present invention.

### [Test Example 1]

The molecular weight of the fraction of a milk-derived basic protein obtained in Example 1 was measured by sodium dodecyl sulfate-polyacrylamide gel electrophoresis (SDS-PAGE), and it was distributed over the range of 3,000 to 80,000.

### [Test Example 2]

The composition of the fraction of a milk-derived basic protein obtained in Example 1 was analyzed. The results are shown in Table 1. As shown in the table, most of the fraction is composed of proteins.

**[Table 1]**

| | |
|---|---|
| Water | 1.06 (wt%) |
| Protein | 96.5 |
| Fat | 0.56 |
| Ash | 0.27 |
| Others | 1.61 |

### [Test Example 3]

The fraction of a milk-derived basic protein obtained in Example 1 was hydrolyzed with 6 N hydrochloric acid at 110°C for 24 hours. After that, the amino acid composition of the resultant was analyzed with an amino acid analyzer (L-8500, manufactured by Hitachi, Ltd.) The results are shown in Table 2. The fraction of a milk-derived basic protein includes 15 wt% or more of basic amino acids in the amino acid composition.

**[Table 2]**

| | |
|---|---|
| Aspartic acid | 10.1 (wt%) |
| Serine | 5.3 |
| Glutamic acid | 12.3 |
| Proline | 4.7 |
| Alanine | 5.7 |
| Leucine | 10.2 |
| Lysine | 8.4 |
| Histidine | 2.6 |
| Arginine | 7.2 |
| Others | 33.5 |

### Example 2

After a column (diameter 5 cm × height 30 cm) filled with 400 g of sulfonated Chitopearl (manufactured by Fuji Spinning Co., Ltd.) as a cation exchange resin was washed with deionized water sufficiently, 40 1 of unsterilized defatted milk (pH 6.7) were passed through the column at a flow rate of 25 ml/min. After that, the column was washed with deionized water sufficiently, and eluted with a 0.02 M carbonate buffer (pH 7.0) containing 2.0 M sodium chloride. Then, the eluted fraction containing lactoperoxidase was adsorbed to an S-Sepharose FF column (manufactured by Amersham Biosciences), and the column was washed with deionized water sufficiently. After the column was equilibrated with 10 mM phosphate buffer (pH 7.0), the adsorbed fraction was eluted with a linear gradient of 0 to 2.0 M sodium chloride by, whereby a fraction containing lactoperoxidase was collected. Then, the fraction containing lactoperoxidase was treated by gel filtration chromatography using HiLoad 16/60 Superdex 75 pg (manufactured by Amersham Biosciences), thereby obtaining 3.0 g of lactoperoxidase. Note that the purity of the thus obtained lactoperoxidase is 94%, and the lactoperoxidase can be used as a prophylactic agent for an autoimmune disease as is.

### Example 3

After a column (diameter 5 cm×height 30 cm) filled with 400 g of sulfonated Chitopearl (manufactured by Fuji Spinning Co., Ltd.) as a cation exchange resin was washed with deionized water sufficiently, 40 1 of unsterilized defatted milk (pH 6.7) were passed through the column at a flow rate of 25 ml/min. After that, the column was washed with deionized water sufficiently, and elution was performed with a 0.02 M carbonate buffer (pH 7.0) containing 2.0 M sodium chloride. Then, the eluted fraction containing lactoferrin was adsorbed to an S-Sepharose FF column (manufactured by Amersham Biosciences), and the column was washed with deionized water sufficiently. After the column was equilibrated with a 10 mM phosphate buffer (pH 7.0), the adsorbed fraction was eluted with a linear gradient of 0 to 2.0 M sodium chloride, whereby a fraction containing lactoferrin was collected. Then, the fraction containing lactoferrin was treated by gel filtration chromatography using HiLoad 16/60 Superdex 75 pg (manufactured by Amersham Biosciences), thereby obtaining 8.0 g of lactoferrin. Note that the purity of the thus obtained lactoferrin is 96%, and the lactoferrin can be used as a prophylactic agent for an autoimmune disease as is.

### [Test Example 4]

### (Effect of inhibiting development of autoimmune disease which induces development of insulin-dependent diabetes mellitus)

By using a type I diabetes mellitus model (NOD) mouse, inhibition effect of the fraction of a milk-derived basic protein, lactoferrin, and lactoperoxidase to autoimmune response which induces the development of insulin-dependent diabetes mellitus was examined.
After NOD mice (CLEA Japan, Inc., 4-week-old) were bred for 1 week with general formula feed, MF feed (manufactured by Oriental Yeast Co., Ltd.), the mice were divided into the following 4 groups (each group includes 7 mice), and bred: control group (MF standard feed); test group T (test feed mixing the fraction of a milk-derived basic protein obtained in Example 1 in MF feed at a content of 0.1 %); test group LP (test feed mixing lactoperoxidase obtained in Example 2 in MF feed at a content of 0.1%); and test group LF (test feed mixing lactoferrin obtained in Example 3 in MF feed at a content of 0.1%). After 38 days of breeding (10-week-old), the sugar concentrations in the urine of 7 mice per group were measured. The standard value was set to 20 mg/dl, which is a normal value of glucose level in urine, and a mouse having a value exceeding the standard value was defined as positive. The sugar concentrations in the urine were measured and it was confirmed whether diabetes mellitus was developed or not. The results are shown in Table 3.

**[Table 3]**

| The number of mice having positive urine sugar value | |
|---|---|
| | (number of positive mice/total number of mice) |
| Control group | 7/7 |
| Test group T | 1/7 |
| Test group LP | 0/7 |
| Test group LF | 5/7 |

As shown in Table 3, in the control group and the test group LF, the urine sugar concentrations exceeded the standard value in 7 mice out of 7 mice and 5 mice out of 7 mice, respectively, and those mice developed diabetes mellitus. On the contrary, only 1 mouse out of 7 mice developed diabetes mellitus in the test group T, and none of 7 mice developed diabetes mellitus in the test group LP. There was no significant difference in the feed ingestion amount and weight change during the test period among the groups. For this reason, it was clarified that the fraction of a milk-derived basic protein and lactoperoxidase has an effect of inhibiting autoimmune response which induces the development of insulin-dependent diabetes mellitus.

### [Test Example 5]

### (Effect of inhibiting autoimmune disease which induces development of swelling)

By using a spontaneous rheumatoid arthritis model (SKG) mouse, inhibition effect of the fraction of a milk-derived basic protein, lactoferrin, and lactoperoxidase to autoimmune response which induces the development of swelling was examined.
After spontaneous rheumatoid arthritis model (SKG) mice (CLEA Japan, Inc., 4-week-old) were bred for 1 week with general formula feed, MF feed (manufactured by Oriental Yeast Co., Ltd.), the mice were divided into the following 4 groups (each group includes 5 mice), and bred: control group (MF standard feed); test group T (test feed mixing the fraction of a milk-derived basic protein obtained in Example 1 in MF feed at a content of 0.1%); test group LP (test feed mixing lactoperoxidase obtained in Example 2 in MF feed at a content of 0.1%); and test group LF (test feed mixing lactoferrin obtained in Example 3 in MF feed at a content of 0.1%). After 38 days of breeding (10-week-old), the development frequency of rheumatoid arthritis was measured after laminarin (L9634, manufactured by Sigma-Aldrich Corporation) was administered intraperitoneally. The evaluation of the development of rheumatoid arthritis was as follows: score for one swelling site in the interdigital osteoarticular; 0.5 score for medium degree swelling in the hand joint and foot joint; and 1.0 score for high degree of swelling. The swelling was measured and an average swelling score per individual was determined. Table 4 shows the results of the development frequency of rheumatoid arthritis.

**[Table 4]**

| Average swelling score (per individual) | |
|---|---|
| Control group | 4.6±1.4 |
| Test group T | 1.8±0.4 |
| Test group LP | 1.1±0.6 |
| Test group LF | 1.5±0.9 |

As shown in Table 4, in the control group, the swelling was developed in all mice and the average swelling score per individual was 4.6. On the contrary, in the test group T, the test group LP, and the test group LF, the swelling was confirmed in only 1 or 2 mice and the average score per individual was about 1.1 to 1.8. There was no significant difference between groups in the feed ingestion amount and weight change during the test period. For this reason, it was clarified that the fraction of a milk-derived basic protein, lactoferrin, and lactoperoxidase had an effect of inhibiting autoimmune response that induces the development of rheumatoid arthritis.

### Example 4

A prophylactic agent for an autoimmune disease having the composition shown in Table 5 was produced by using the powdered fraction of a milk-derived basic protein obtained in Example 1 according to a conventional method.

**[Table 5]**

| | |
|---|---|
| Water-containing crystal glucose | 81.1 (wt%) |
| Soybean protein | 12 |
| Mineral mixture | 5 |
| Sugar ester | 1 |
| Flavor | 0.5 |
| Powdered fraction of milk-derived basic protein (Example 1) | 0.4 |

### Example 5

A prophylactic agent for an autoimmune disease, which has the composition shown in Table 6 and has an effect of inhibiting autoimmune response that induces the development of rheumatoid arthritis, was produced according to a conventional method by using the lactoperoxidase powder obtained in Example 2.

**[Table 6]**

| | |
|---|---|
| Water-containing crystal glucose | 80.3 (wt%) |
| Soybean protein | 13 |
| Mineral mixture | 5 |
| Sugar ester | 1 |
| Flavor | 0.5 |
| Lactoperoxidase powder (Example 2) | 0.2 |

### Example 6

A prophylactic agent for an autoimmune disease having the composition shown in Table 7 was produced according to a conventional method by using the lactoferrin powder obtained in Example 3.

**[Table 7]**

| | |
|---|---|
| Water-containing crystal glucose | 80.3 (wt%) |
| Soybean protein | 13 |
| Mineral mixture | 5 |
| Sugar ester | 1 |
| Flavor | 0.5 |
| Lactoferrin powder (Example 3) | 0.2 |

## Claims

1. A prophylactic agent for an autoimmune disease, comprising a fraction of a milk-derived basic protein as an active ingredient.

2. A prophylactic agent for an autoimmune disease according to Claim 1, wherein the milk-derived basic protein comprises lactoperoxidase and/or lactoferrin.

3. A prophylactic agent for an autoimmune disease, comprising a fraction of a milk-derived basic protein as an active ingredient, wherein the autoimmune disease is type I diabetes mellitus.

4. A prophylactic agent for an autoimmune disease according to Claim 3, wherein the milk-derived basic protein comprises lactoperoxidase.

5. A prophylactic agent for an autoimmune disease, comprising a fraction of a milk-derived basic protein as an active ingredient, wherein the autoimmune disease is rheumatoid arthritis.

6. A prophylactic agent for an autoimmune disease according to Claim 5, wherein the milk-derived basic protein comprises lactoperoxidase and/or lactoferrin.
